# EUROPEAN PATENT APPLICATION

(11) **EP 4 524 253 A1**
(43) Date of publication of application: **19.03.2025**
(21) Application number: 23197336.3
(22) Date of filing: 14.09.2023
(51) Int. Cl.: C12P 19/14, D21C 1/02, C13K 1/02, C13K 1/00

(54) **METHOD OF OBTAINING A SOLUTION CONTAINING FREE SUGARS FROM A SECOND-GENERATION FEEDSTOCK AND SOLUTION OBTAINED THEREBY**

(71) Applicant: Fibers365 GmbH, 73252 Lenningen (DE)
(72) Inventor: LABRIOLA, Leticia, Lenningen (DE); DAUSER, Hermann Josef, Lenningen (DE); CLAUSS, Manuel Maximilian, Lenningen (DE)
(74) Representative: Pritzlaff, Stefanie Lydia

(57) **Abstract**

A method of obtaining a solution containing free sugars from a second-generation feedstock, the method comprising the steps of: providing biomass from a second-generation feedstock; at least once pre-treating the biomass by steam explosion, the steam exploded biomass comprising a liquid portion and a solids portion; at least once adding enzymes to the steam exploded biomass to increase an amount of free sugar via enzymatic digestion over a predetermined time, at a predetermined pH value and at a predetermined temperature; and obtaining the solution by at least partially removing the solids portion from the enzymatically digested biomass.

## Description

### Field of the invention

The present invention is directed at obtaining a solution containing free sugars from a second-generation feedstock for biotechnological applications based on annually renewable biomass.

Solutions as obtained by the invention may have a particular use in biotechnological applications standing in context with bacterial growth. Their production may precede the production of biodegradable bioplastics such as Polylactid Acids (PLAs), Polyhydroxyalkanoates (PHAs) etc. Further applications may be found in the field of bio combustibles, for instance in context with the production of ethanol, biogas etc. It may also be found in context with yeast growth and in the production of lipids, fatty acids, terpenes and/or products from their respective metabolism.

### Background of the invention (and prior art)

From the prior art such as US 10 584 298 B2, for instance methods for manufacturing biomass based fuel are known aiming to reduce chemical and/or mechanical effects of flue gas on heat transfer surfaces. The method comprises providing biomass and steam into a reactor, maintaining said biomass and said steam simultaneously in the reactor in a pressure of at least 10 bar and at a temperature from 180 °C to 250 °C for at least 2 minutes and decreasing the pressure in the reactor and / or conveying biomass out of the reactor such that the pressure of the environment of the biomass decreases below 5 bar, to produce steam-exploded biomass. The method of US 10 584 298 B2 further comprises adding some combustion additive to the biomass and / or the steam-exploded biomass.

Moreover, EP 2 177 280 B1 discloses an apparatus for discontinuous hydrolysis of organic substrates, in particular energy crops, as well as vegetable or animal waste, which comprises a pre-conditioning tank for receiving solid and/or liquid organic substances, and a charging device for filling a hydrolyzer with the substrates from the tank, and an expansion device having a flash tank for depressurizing the substrates after treatment in said hydrolyzer. The expansion device is provided with a steam separator preceding the flash tank and placed downstream of a pressure baffle, a device for directly subjecting the input of organic substrates to superheated steam from the steam separator being furnished. EP 2 177 280 B1 defines "steam explosion" as a technical process in which the starting material is heated to up to 300°C, preferably 150°C to 200°C, and then pressurized under an overpressure of approx. 3 bar to 20 bar. After a certain dwell time, the substrate is spontaneously depressurized to about atmospheric pressure. Such impact relaxation results in a complete disintegration of the cell substance. The entire organic substance is then liquefied and made available for further processing.

While known systems that apply hydrothermal hydrolysis generally work with temperatures around 185 to 200 °C, tests show that the final products have an increased concentration of furfural and hydroxy-methyl-furfural. These compounds, however, are inhibiting the growth of microorganisms such as bacteria, yeast, etc. which are most useful for further applications of the obtained product. Moreover, known systems and methods generally use wood or wooden byproducts, are therefore limited when it comes to material choices and are consequently more dependent on specific harvest seasons.

One of the primary challenges with second-generation feedstocks is the intrinsic material stability of lignocellulosic materials. Unlike first-generation feedstocks (e.g., those derived from beets, corn, sugarcane etc.), which contain easily accessible sugars, lignocellulosic biomass is composed of complex structures that require pre-treatment and enzymatic hydrolysis to release the sugars contained thereon. Developing efficient and cost-effective pre-treatment methods and robust enzymatic cocktails for biomass deconstruction is therefore a key challenge in the adoption of 2G feedstocks at scale.

The task of the present invention is to overcome the disadvantages described above, and in particular, to provide methods and systems that specifically allow tailoring a feedstock according to uniquely required technical specifications and applications.

### Summary of the invention

The above-mentioned task and further problems are solved by a method of obtaining a solution containing free sugars from a second-generation feedstock and a solution in accordance with the independent claims. The dependent claims relate to preferred embodiments of the invention.

According to one aspect of the present disclosure, a method of obtaining a solution containing free sugars from a second-generation feedstock is provided, the method comprising the steps of: providing biomass from a second-generation feedstock, at least once pre-treating the biomass by steam explosion, the steam exploded biomass comprising a liquid portion and a solids portion, at least once adding enzymes to the steam exploded biomass to increase an amount of free sugar via enzymatic digestion over a predetermined time, at a predetermined pH value and at a predetermined temperature, and obtaining the solution by at least partially removing the solids portion from the enzymatically digested biomass.

Optionally, the provided biomass from the second-generation feedstock may comprise up to 25 wt% of dry matter and up to 75 wt% of water before the pre-treatment by steam explosion. Alternatively, the provided biomass from the second-generation feedstock may comprise up to 30 wt% of dry matter and up to 70 wt% of water before the pre-treatment by steam explosion, or the provided biomass from the second-generation feedstock may comprise up to 35 wt% of dry matter and up to 65 wt% of water before the pre-treatment by steam explosion. In all optional cases, the provided biomass from the second-generation feedstock may be a biomass that optionally may have been adapted to steam explosion operating conditions. Depending on the kind of second generation feedstock, original biomass (e.g. straw) has a dry matter content typically between 75 and 95%, while other resources may be semi-dry from the beginning, like some silage between 20 and 40 % dry matter. A semi-dry feedstock may be treated without further watering/wetting, while a dry feedstock demands mixing with liquid (e.g. water) to reach a preferred dry matter content.

Moreover, the method may optionally further comprise a step of at least once adding further biomass during the enzymatic digestion, wherein preferably the further biomass is derived from the same and/or from a different biomass than the steam exploded biomass; and/or wherein preferably the further biomass is pre-treated by steam explosion.

Optionally, the provided biomass and/or the further biomass may be a lignocellulosic biomass and/or a wood-free biomass, respectively, wherein the provided biomass and/or the further biomass is derived from a renewable biomass, preferably from an annually renewable biomass, respectively, wherein the provided biomass and/or the further biomass comprises: agricultural waste and/or at least one agricultural by-product, preferably at least one of wheat straw, tobacco stems, hemp, rice straw, thistle, paludi culture, one or more different types of grasses, cocoa husk, sunflower husk, apple pomace, maize, arundo donax or a combination thereof, and/or wherein the provided biomass and/or the further biomass is derived from one single source or is a composition of various plants and/or plant parts, respectively.

Furthermore, the step of pre-treating the biomass by steam explosion may optionally comprise at least one of the following steps: steam exploding the biomass under a predetermined amount of pressure, preferably between 5 bar to 10 bar, more preferably between 7 and 9 bar; steam exploding the biomass for a predetermined amount of time, preferably between 10 s to 30 min, preferably between 10 and 15 min; and steam exploding the biomass at a predetermined temperature, preferably in a range of 150 and 180°C, more preferably in a range of 165 to 175 °C. The step of pre-treating the biomass by steam explosion may optionally be repeated at least once, with intermediate relaxation of pressure and temperature in between the repetitions. This part of the process is called a thermo-pressure hydrolysis (TPH). The relaxation process of the biomass being pre-treated, in combination with a discharge of treated biomass from a respective vessel ("steam cooker"), is called steam explosion. The pressure drop is optionally at least 4 bar, preferably at least 5 bar. Each relaxation of pressure and temperature lasts optionally for 10 to 15 minutes.

The predetermined temperature for the enzymatic digestion is optionally a range between 45 to 49 °C, preferably about 47 °C; and/or the predetermined pH value optionally lies between 4.0 and 5.0.

Moreover, the liquid portion of the steam exploded biomass may optionally be kept or it may be at least partially replaced by any liquid, preferably water, hydrolysate or a mixture thereof before the enzymatic digestion.

In addition, the method may optionally further comprise a step of concentrating the obtained solution based on a predefined sugar concentration, the predefined sugar concentration preferably being a predefined carbohydrate concentration, the predefined carbohydrate concentration preferably being a monosaccharaide concentration and/or a disaccharide concentration, and/or the predefined carbohydrate concentration most preferably comprising at least one of the following: glucose, xylose, galactose, mannose, arabinose and cellobiose. In this context, the predefined glucose concentration may optionally lie between 50 and 600 g/L.

Further, the steam explosion may additionally comprise a step of heating the biomass under a predefined pressure to a temperature below 180 °C, preferably to a temperature between 165 to 175 °C, and more preferably for about 10 to 15 min; and then a step of suddenly releasing pressure.

Preferably, no chemical products, such as buffers, more preferably acids and/or bases, are added during the step of pre-treating.

Optionally, the step of adding enzymes comprises adding cellulases and/or hemicellulases or a blend thereof. Optionally, such blend may additionally comprise laccase.

Also optionally, the step of at least partially removing comprises separating the liquid portion from the non-digested solids portion of the solution by centrifuging and/or filtering; and, preferably, the step of filtering is a multi-stage filtering. The remaining non-digested solids portion is optionally less than 1 wt%; wherein preferably said 1 wt% mainly comprise purified lignin; and/or wherein preferably a total concentration of free carbohydrates lies between 50 to 100 g/L. Again optionally in this context, the method may further comprise a step of concentrating the centrifuged and/or filtered solution based on a further predefined total content of free carbohydrates, the total concentration of free carbohydrates after the step of concentrating preferably lying between 300 and 600 g/L.

Further, the method may comprise a step of concentrating the obtained solution based on a predefined concentration of free carbohydrates, wherein the predefined concentration of free carbohydrates preferably lies between 300 and 600 g/L
According to another aspect of the present disclosure, a solution is provided containing at least 5 wt % or at least 50 to 100 g/L, preferably up to 600 g/L free sugars and obtained by the method of obtaining a solution containing free sugars from a second-generation feedstock in accordance with the present disclosure. The solution comprises carbohydrates, proteins, organic acids, mineral salts and small non-inhibitory concentrations of furfural and 5-hydroxyfurfural (HMF), preferably below or equal to 0.71 g/L, most preferably in the range of 1.18 and 1.26 g/L for furfural and in the range of 0.3 -0.4 g/L for HMF.

The solution of the present invention may optionally further comprise lactic acid depending on at least one of the following: the biomass provided from the second-generation feedstock, the pre-treatment of the provided biomass, the further biomass added during enzymatic digestion and the pre-treatment of the further biomass.

In this context, the solution may optionally be further defined as to fulfill at least one of the following conditions: the carbohydrates comprise at least one of the following: glucose, xylose, galactose, mannose, arabinose and cellobiose; the organic acids comprise at least one of the following: acetic, formic, levulinic and propionic; and the mineral salts comprise at least one of the following: sodium, potassium or calcium nitrate, sodium, potassium or calcium phosphates and sodium, potassium or calcium silicates.

The method of the present invention may advantageously be implemented in decentralized steam explosion units coupled to biogas production plants at farm level. The energy necessary for the process as a whole can be fully covered by the biogas plant. It is specifically applicable not only to regional biomass, but may at the same time take into account specific timing issues that may come with natural conditions like harvest timing etc. The specific method of the present invention makes potential waste treatment unnecessary. Also, it is specifically directed to non-food biomass that is obtained either from agricultural by-products or from by-products of food generation processes that do not compete with land for food generation. By utilizing agricultural residues, the solutions of the present invention have the potential to reduce greenhouse gas emissions by replacing fossil-based and de-forestation-based ingredients and consumer products. In addition, the present invention allows for extraction of functional cellulosic fibers, lignin and sugars from multi feedstock non-wood biomass. These sugars can be even further valorized by producing high-value products via fermentation for various industrial applications. Moreover, another advantage of using a non-wood biomass in the steam explosion process lies in the fact that, unlike wood, annual plants can close the CO₂ loop in only one year. Thus, they contribute in both a faster and more efficient manner than wood to diminishing worldwide production of greenhouse gases.

### Brief description of the drawings

The invention, as well as further details and advantages thereof, are explained below with reference to preferred examples of embodiments with reference to the figures. Showing:
Fig. 1 shows a timeline of a first enzymatic digestion experiment (Experiment 1);
Fig. 2 shows three evaluation charts relating to the development of total free sugars (mg/mL), total free glucose (mg/mL) and total free xylose (mg/mL) over time (h) in the first experiment of Fig. 1;
Fig. 3 shows a timeline of a second enzymatic digestion experiment (Experiment 2);
Figs. 4A and 4B show results of a particle size analysis of the substrate used in the second experiment (steam exploded wheat straw WSSE versus non-steam exploded wheat straw WSnonSE);
Fig. 5 shows three evaluation charts relating to the development of total free sugars (mg/mL), total free glucose (mg/mL) and total free xylose (mg/mL) over time (h) in the second experiment of Fig. 3;
Fig. 6 shows a comparison of specific results from the first and the second experiment with respect to total free sugars (mg/mL), total free glucose (mg/mL) and total free xylose (mg/mL) over time (h);
Fig. 7 shows a timeline of a third enzymatic digestion experiment (Experiment 3);
Fig. 8 shows results of a particle size analysis of the substrate used in the third experiment (steam exploded wheat straw WSSE versus steam exploded canary grass, RGSE);
Fig. 9 shows three evaluation charts relating to the development of total free sugars (mg/mL), total free glucose (mg/mL) and total free xylose (mg/ml) over time (h) in the third experiment of Fig. 8.

### Detailed description

In the following, directional terms such as "top", "bottom", "right" and "left" or similar indications refer to orientations or arrangements shown in the figures to describe the respective embodiments. While such expressions may show preferred arrangements, they are not to be understood in a limiting sense.

Further, the terms "substantially", "approximately", "about" and similar expressions mean that deviations of +/-10%, preferably +/-5%, from said values are permissible.

The term "feedstock" encompasses in this context raw materials, materials made usable or enhanced materials for industrial processing. In general, first generation feedstocks such as corn, wheat, sugarcane, potato, sugar beet, rice or plant oil are rich in carbohydrate and/or fatty acids and can be consumed by humans and animals. First generation feedstocks are known to be the most efficient way to produce for instance bioplastics as they require less land to grow, and have a higher yield and better performance than other feedstock generations. Moreover, second generation feedstocks generally refer to crops and plants not suitable for human consumption (food) or animal consumption (feed). Second generation feedstocks can be either non-food crops (cellulosic and/or lignocellulosic feedstock) or waste materials from first generation feedstocks (e.g. waste vegetable oil). Examples of second generation feedstock include: wood, short-rotation crops such as poplar, willow or miscanthus (elephant grass), wheat straw, bagasse, corncobs, palm fruit bunches and sweet grasses.

In the present invention, biomass based on second-generation feedstock is being enhanced by specific combinations of at least one pre-treatment together with a specific chemical-free processing. Preferably, such pre-treatment comprises a steam explosion treatment.

The term "biomass" in this context relates to agricultural waste such as wheat straw, tobacco stems, hemp, rice straw, thistle, paludi culture, miscanthus, different types of grasses, cocoa husk, sunflower husk, apple pomace, maize (especially the rest of the plant after extracting the corn), arundo donax etc. The biomass used for the present invention is not limited to said specific plants, rather they are listed here as examples the method of the present invention can be implemented with. Biomass as used in the present invention may be derived from one single source or may be a composition of various plants and/or plant parts.

The term "derivative" encompasses products or byproducts obtained by methods of the present invention based on feedstock, and particularly on second generation feedstock as explained above.

The present invention relates to methods of obtaining a solution containing free sugars from a second-generation feedstock. Initially, biomass from a second-generation feedstock is at least once pre-treated by steam explosion. As various Experiments that will be discussed below show, this process is crucial when it comes to the quality of the final solution to be obtained. It is preferably performed in mild conditions of temperature, i.e. about 165 to 175 °C for about 10 to 15 min. Preferably, no chemical products such as acids or bases are added during this pretreatment of the biomass. The steam exploded biomass comprises a liquid portion and a solids portion.

Since each biomass reacts differently during each step of the method of the present invention (see in this context Examples 1 to 5 below), these parameters may vary already when it comes to the step of pre-treating the biomass by steam explosion. Therefore, this step may optionally comprise at least one of steam exploding the biomass under a predetermined amount of pressure, preferably between 7 and 9 bar, steam exploding the biomass for a predetermined amount of time, preferably between 10 and 15 min, and steam exploding the biomass at a predetermined temperature, preferably in the range of 165 to 175 °C.

In the following, Table 1 provides exemplary value combinations for the parameters temperature, pressure and residence time that may be used for the present invention, for instance in context with wheat straw (WS) but also applicable to other biomass:

**Table 1**

| Temperature (°C) +/- 2 °C | Pressure (bar) | Residence time (min) |
|---|---|---|
| 165 | 7,74 | 10 |
| 168 | 8 | 10 |
| 170 | 8,87 | 10 |
| 172 | 9 | 10 |

Table 1 especially takes into account that for instance cellulose should not be submitted to more than 195 °C otherwise it would disintegrate and/or burn. Furthermore, hemicellulose starts dissolving over 110 °C. Over 150 °C it starts disintegrating, burning and forming inhibitors. Finally, lignin starts melting from 150 °C on and the probability increases that it burns and starts forming inhibitors. Consequently, depending on the substrate / biomass, the temperature range for steam explosion in the context of the invention may lie between 150 °C and 180°C, preferably in the range of 165 °C to 175 °C.

After the steam explosion, at least once enzymes are added to the steam exploded biomass to increase an amount of free sugar via enzymatic digestion over a predetermined time, at a predetermined pH value and at a predetermined temperature. It may optionally useful to also predetermine an amount or percentage of solid enzymatic substrate that will and/or may remain in the final solution.

A practical aspect when it comes to predefining characteristics of both biomass and steam explosion relates to the original quality of the biomass used. It may be advantageous to use a liquids portion or stream coming from a steam exploded biomass comprising a blend of cellulases and hemicellulases in the presence of fibers of rather marginal quality that are not suitable for further use, for instance in the paper or packaging industry. Depending on parameters such as quality, amount etc. of the fibers present in such a blend, the solids portion may be partially filtered out of before the enzymatic digestion process is started.

After the enzymatic digestion, the solution is obtained by at least partially removing the solids portion from the enzymatically digested biomass.

It is one specific advantage that the solution of the present invention may be specifically tailored regarding its downstream further processing process. It is noted that the specific downstream processes are not part of the invention anymore, but may clearly be of help or even crucial when it comes to predefining the parameters for the method of the invention in order to arrive at a specifically tailored solution. Such tailoring comprises not only the control of proportions between liquid and solids portion, but is especially related to the amount and type of total free sugars in combination with and/or in the specific absence of further substances in the same solution.

Depending on criteria the final solution has to fulfill for a specific downstream process, there are various options to treat the obtained solutions. It can for instance be centrifuged for separating a resulting liquid phase from non-digested solids (mainly constituted by purified lignin). A resulting total free carbohydrate concentration lies for instance between 50 and 100 g/L.

Optionally and depending on a client's specifications, the obtained solution can additionally or alternatively be submitted to a concentration step for increasing its content of total free carbohydrates. A resulting total free carbohydrate concentration afterwards lies for instance between 300 and 600 g/L.

A solution in accordance with the present invention contains at least 5 % or at least 50 to 100 g/L, preferably up to 600 g/L free sugars. The solution comprises carbohydrates, proteins, organic acids, mineral salts and small non-inhibitory concentrations of furfural and 5-hydroxymethylfurfural (HMF), preferably below or equal to 0.71 g/L, most preferably in the range of 1.18 and 1.26 g/L for furfural and in the range of 0.3 -0.4 g/L for HMF.

The carbohydrates may comprise at least one of the following: glucose, xylose, galactose, mannose, arabinose and cellobiose. The organic acids may comprise at least one of the following: acetic, formic, levulinic and propionic. The mineral salts may comprise at least one of the following:
sodium, potassium or calcium nitrate, sodium, potassium or calcium phosphates and sodium, potassium or calcium silicates.

Depending on at least one of the specific biomass provided from the second-generation feedstock, the specific pre-treatment of the provided biomass, further biomass optionally added during enzymatic digestion and an optional pre-treatment of this further biomass may result in the solution optionally also comprising lactic acid.

In the context of inhibitors as such or inhibiting substance combinations, reference is made to Karolin Dietrich, Marie-Josée Dumont, Timothy Schwinghamer, Valerie Orsat, and Luis F. Del Rio: "A model study to assess softwood hemicellulose hydrolysates as carbon source for PHB production in Paraburkholderia sacchari IPT 101", in: Biomacromolecules, University of Florida Libraries, 2017. The authors tested the potential of softwood hemicellulose hydrolysates of being used as a carbon source together with potentially inhibitory lignocellulose degradation derived products such as acetic acid, 5-hydroxymethylfurfural, furfural and vanillin by fermenting with the model strain *Paraburkholderia sacchari IPT 101.* This study pointed that organic acids, furans and phenols can act as growth inhibitors as well as substrates. Furthermore, as a general conclusion, no inhibitor alone causes complete growth inhibition, indicating that individual toxicity can be overcome. However, specific inhibitor mixtures may stop growth entirely.

Further reference is made to Hongzhi Huang, Xinyan Guo, Dongmin Li, Mengmeng Liu, Jiafang Wu, Haiyu Ren: "Identification of crucial yeast inhibitors in bio-ethanol and improvement", in: Bioresource Technology 102 (2011) 7486-7493, indicating that weak acids were identified as main yeast growth or fermentation inhibitors, while phenols and aldehyde contribute to the inhibition to a lower degree. Main weak acids found in hydrolysates are acetic acid and formic acid, which display critical levels in yeast growth or fermentation inhibition at concentrations of 6 and 4 g/L, respectively (see in this context Table 1 of this document). In particular, formic acid did not show any negative effect on fermentation at low concentrations (1-3 g/L), where a slight positive effect was even seen compared to the control, whereas obvious inhibition was observed at a concentration of 4 g/L. Ethanol production was almost completely inhibited at concentrations of 6 and 8 g/L. Acetic acid showed a similar trend to that of formic acid, as it did not inhibit ethanol production at low concentrations (1-4 g/L), while high levels of acetic acid (6 g/L) resulted in significant inhibition. Formic acid was generally observed to be a stronger inhibitor than acetic acid.

In view of the above, the solution of the present invention may be tailored so as to comprise the following exemplary amounts of acetate, HMF, furfural, levulinic acid, formiate and soluble lignin:
Acetate: 1.51 g/L, 1.46 g/L, 2.14 g/L and 1.2 g/L measured in four different experiments using steam exploded wheat straw; 1.8 g/L, 1.78 g/L measured in two different experiments using steam exploded canary grass (scientific name: *Phalaris)* fibers;
HMF (furfural derivative inhibitor, especially 5-hydroxymethylfurfural): 0.31-0.33 g/L measured in different experiments using steam exploded wheat straw;
Furfural: 1.18-1.26 g/L measured in different experiments using steam exploded wheat straw;
Vanillin: Not detectable / below detection limit (detection limit DL: 0.01g/L) in different experiments using steam exploded wheat straw;
Levulinic acid: Not detectable / below detection limit (DL: 0.01g/L) in different experiments using steam exploded wheat straw;
Formiate: 0.04 g/L, 0.04 g/L, 0.02 g/L, 0.12 g/L, 0.1 g/L, 0.05 g/L measured in six different experiments using steam exploded wheat straw;
Soluble lignin: 2.64 - 4.4 g/L (measured in different experiments using steam exploded wheat straw), 2.64 - 3.08 g/L (measured in different experiments using steam exploded read grass fibers), 2.64 g/L (measured in different experiments using steam exploded reed grass), 3.3 g/L (measured in different experiments using steam exploded sweet grass).

Compared to known biomass applications, for instance wherein the biomass is not submitted to steam explosion, second-generation feedstock derivatives of the present invention have a significantly increased content of free carbohydrates allowing for a specifically high degree of microorganisms proliferation.

In the following, two examples (Examples 1 and 2) of second generation feedstock derivatives in accordance with the present invention are being provided. For both Examples 1 and 2, a liquid feedstock derivative from wheat straw was produced as an aqueous media comprising a high number of nutrients extracted from annual plants such as wheat straw by means of a steam explosion process. The liquid substrate itself can again function as a feedstock for bio energy / biogas plants and replaces food and husbandry-based feedstock. Moreover, valuable organic acids and a wide variety of C5 and C6 sugars serve as platform chemicals for a fermentation to bio-based products such as biopolymers PLA or PHA. Because of a relatively mild extraction process at about 165-175°C for about 10 to 15 minutes, the formation of C6 or C5 sugars degradation products, other than for example acetic and formic acid, is marginal and not negatively affecting fermentation processes.

### Example 1: Liquid second generation feedstock derivative from straw

| Physical appearance | Brown liquid with less than 0.1% solid matter of total weight) |
|---|---|
| *pH (aqueous)* | 4.5 |
| Carbohydrates (g/L) [wt%] | 9.0 wt%; 90 g/L. |

The wheat straw derived sample of Example 1 was analyzed to determine the concentration of free monosaccharides and detectable disaccharides in solution. This analysis was carried out at the Fibers365 GmbH quality assurance laboratory by high performance liquid chromatography (HPLC) according to the NREL standard (NREL/TP-510-42618).

**Table 1. Monosaccharides / Disaccharides in Solution**

| | **Monosaccharides/ Disaccharides** | **Final sample (mg/mL)** | **% of total Monosaccharides / Disaccharides** |
|---|---|---|---|
| Glucose | | 37± 3 | 42.6 ± 0.5 |
| Xylose | | 45.5 ± 0.7 | 51.8 ± 0.3 |
| Galactose | | 3.1± 0.7 | 3.4 ± 0.7 |
| Arabinose | | 1.4 ± 0.3 | 0.5 ± 0.1 |
| Mannose | | 2.3 ± 0.3 | 2.7 ± 0.9 |
| Cellobiose | | 1.2 ± 0.7 | 1.4 ± 0.5 |
| **Total** | | **90 ± 5** | **100 ± 3** |

### Example 2: Liquid second generation feedstock derivative from wheat straw

| Physical appearance | Brown liquid with less than 0.1% solid matter of total weight) |
|---|---|
| *pH (aqueous)* | 4.5 |
| Carbohydrates (g/L) [wt%] | 32.6 wt%; 326 g/L. |

The wheat straw derived sample of Example 2 was analyzed to determine the concentration of free monosaccharides and detectable disaccharides in solution. This analysis was carried out at the Fibers365 GmbH quality assurance laboratory by high performance liquid chromatography (HPLC) according to the NREL standard (NREL/TP-510-42618).

**Table 2. Monosaccharides / Disaccharides in Solution**

| | **Monosaccharides / Disaccharides** | **Final sample (mg/mL)** | **% of total Monosaccharides / Disaccharides** |
|---|---|---|---|
| Glucose | | 221± 3 | 67.6 ± 0.5 |
| Xylose | | 89.5 ± 0.7 | 27.4 ± 0.3 |
| Galactose | | <D.L | 0% |
| Arabinose | | 3.4 ± 0.3 | 0.50 ± 0.05 |
| Mannose | | 8 ± 2 | 2.3 ± 0.9 |
| Cellobiose | | 7 ± 1 | 2.2 ± 0.5 |
| **Total** | | **326 ± 2** | **100** |

| | | | |
|---|---|---|---|
| **<D.L.: below detection limit.** | | | |

In the following experiments (Experiments 1 to 3), it was tested whether an optional addition of fibers during the enzymatic digestion helps to increase the free sugar content or concentration in the liquid portion / aqueous phase, i.e. finally of the second-generation feedstock derivative to be obtained.

### Experiment 1: Enzymatic Digestion 1

In the following first enzymatic digestion experiment (Experiment 1), it was evaluated whether an addition of lignocellulose or lignin-containing microfibers (LCMF) or of ground steam exploded fibers during the step of enzymatic digestion is helpful for increasing the free sugar concentration in the liquid hydrolysates that were obtained after steam explosion.

Experiment 1 was based on three individual Samples 1 to 3 taken from the same hydrolysate. Moreover, as negative control, an additional reference sample of 1L of only the liquid hydrolysate (V47-WSSE-26012023, the same used in all the experiments) was prepared.

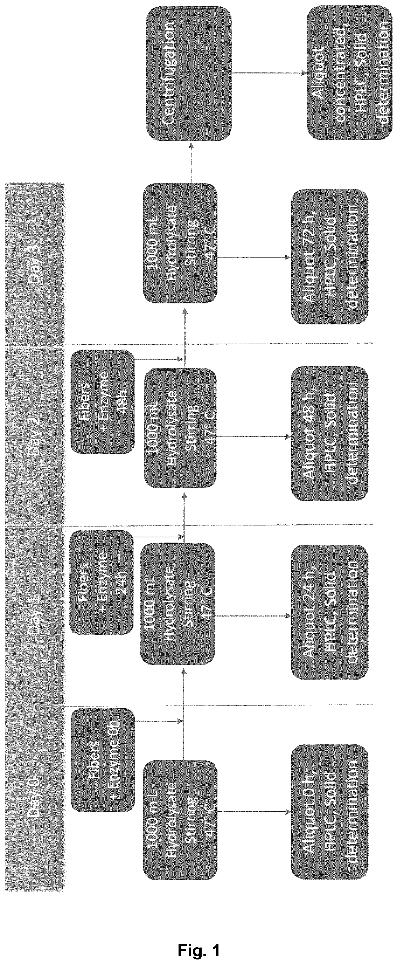

As depicted in Fig. 1 showing a timeline of Experiment 1, on "Day 0", at time 0 h, Samples 1 to 3 were started by adding steam exploded fibers, in particular 70g/L (6 wt%) of dried wheat straw pulp V40 in V47-WSSE-26012023 as hydrolysate to each sample. At the same time, an enzymatic cocktail (Novozymes Cellic Ctec3HS: 4 mL/flask, hydrolysate: V47-WSSE-26012023) were added to all samples, the total volume remained at 1000 ml of hydrolysate, all three samples were stirred at 47°C. Novozymes Cellic Ctec3HS is a specific enzyme blend that converts lignocellulosic materials to fermentable sugars. In this context, it offers both process and substrate versatility and is specifically useful for process optimization and higher concentration yields.

After 24h of digestion ("Day 1"), fibers and enzymes (Novozymes Cellic Ctec3HS: 3 mL/flask), were added to the same hydrolysate (V47-WSSE-26012023) as stated in the following:
Sample 1: 90 g of dried wheat straw pulp V40 (13.8 wt%);
Sample 2: 90 g of dried wheat straw pulp V40 (13.8 wt%);
Sample 3: 90 g of dried wheat straw pulp V40 (13.8 wt%);
and, as depicted in Fig. 1, the total volume remained at 1000 mL of hydrolysate. The samples were incubated under agitation at 47°C.

After 48h of digestion ("Day 2"), fibers and a blend of cellulases (Novozymes Cellic Ctec3HS: 3 mL/flask), were added to the same hydrolysate (V47-WSSE-26012023) as stated below:
Sample 1: 32.77 g of dried wheat straw pulp V40 (16.2 wt%);
Sample 2: 52.25 g of dried wheat straw pulp V40 (17.5 wt%);
Sample 3: 61.87 g of dried wheat straw pulp V40 (18.1 wt%);
and, as depicted in Fig. 1, the total volume remained at 1000 ml of hydrolysate. The samples were incubated under agitation at 47°C.

After 72h of digestion ("Day 3"), Experiment 1 was terminated and samples were taken for the determination of the final free carbohydrate concentration in the final feedstock. In summary the following total amounts of dried ground fibers were added during the whole Experiment 1:
Sample 1: 192.77 g of dried wheat straw pulp V40 (16.2 wt%);
Sample 2: 212.25 g of dried wheat straw pulp V40 (17.5 wt%);
Sample 3: 221.87 g of dried wheat straw pulp V40 (18.1 wt%).

As depicted in Fig. 1, aliquots were obtained at 0 h, 24 h, 48 h and 72 h. Free contents of mono- and disaccharides were determined in each aliquot by using high performance liquid chromatography (HPLC) according to NREL standard procedure (NREL/TP-510-42618). Further, respective dry matter contents at 105°C were also evaluated in the same aliquots obtained at all the time points described in Fig.1 (i.e. at 0, 24, 48 and 72h).

As depicted in Fig. 1, at "Day 3", the hydrolysate was submitted to a centrifugation step. Corresponding supernatant and pellet fractions were collected in different containers. Free mono- and disaccharides concentrations were assessed in the supernatant as previously stated. Dry matter contents were determined both in the supernatant and pellet fractions.

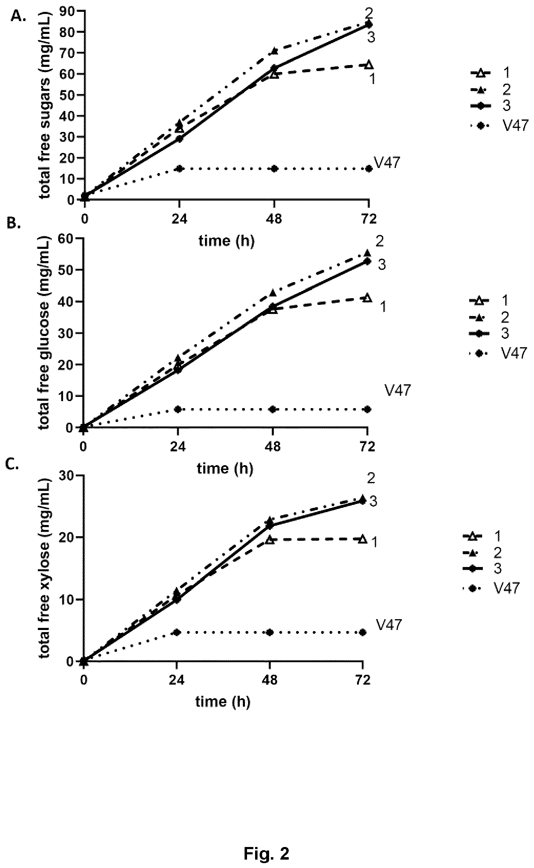

Fig. 2, and especially charts A to C, show concentration increases of total free sugars (chart A), total free glucose (chart B) and total free xylose (chart C) over a digestion time (h) obtained in an experiment design according to Fig.1. In Fig. 2, Sample 1 is designated with reference sign 1, Sample 2 is designated with reference sign 2 and Sample 3 is designated with reference sign 3. Moreover, reference sign V47 designates a liquid hydrolysate obtained after steam explosion without any fiber addition submitted to the same enzymatic conditions as a negative control.

In Experiment 1, agitation of Sample 1 was weaker after 48h than in Samples 2 and 3. This suboptimal agitation jeopardized the subsequent last addition of higher amounts of fibers in Sample 1 finally leading to the lowest yield of the reaction in terms of free total, glucose or xylose concentration after 72h of enzymatic digestion, as depicted in charts A to C of Fig 2.

Agitation as a general parameter is used to promote the effective interaction between the enzyme and the substrate promoting maximal catalytic activity which in this case would be maximal hydrolysis of glucose and xylose from cellulose and hemicellulose present in the fibers. In case of high consistency in the suspension this process is restricted, therefore compromising the yield of the reaction.

To overcome this specific problem, instead of all at once, smaller amounts of additional fibers from the same or from a different second-generation feedstock may be added at multiple, sequential points in time during the enzymatic digestion process. Moreover, additional agitation steps may optionally be carried out during the enzymatic digestion process. Both methods, alone or in combination, may aid in determining when to stop the process of enzymatic digestion, as will be exemplarily shown in the following experiments.

### Experiment 2: Enzymatic Digestion 2

In the following second enzymatic digestion experiment (Experiment 2), two analyzes were carried out. The first focused on the reproducibility of results when different batches of steam exploded wheat straw were submitted to the same process of enzymatic digestion. The second one focused on establishing an influence of steam explosion concerning the concentration of free sugars in a final feedstock.

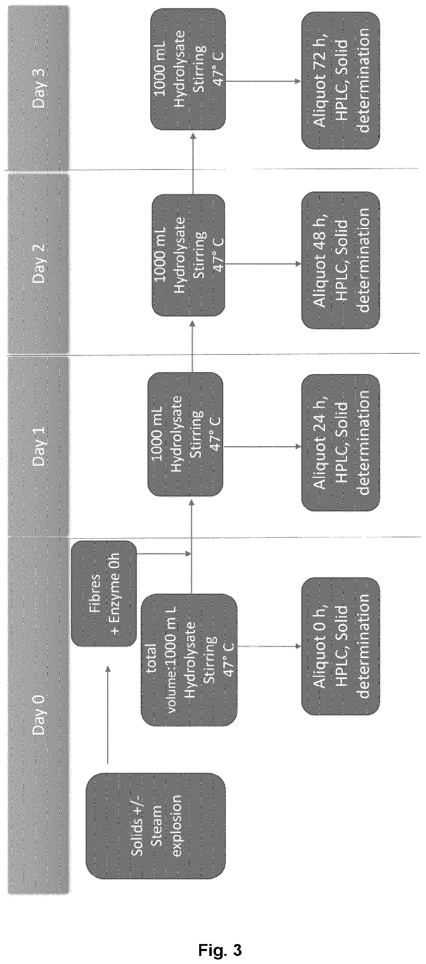

For the first analysis, two different batches of steam exploded wheat straw fibers, particularly dried and ground wheat straw pulp V40A-2023 and WSSE-Italy 2022, were submitted to the enzymatic digestion protocol depicted in Fig. 1. For the second analysis, dried and ground wheat straw fibers from Italy 2022 submitted or not to steam explosion, WSSE-Italy 2022 and non-SE wheat straw (WSnonSE), respectively, were submitted to the enzymatic digestion following a protocol depicted in Fig. 3.

During Experiment 2, only one addition of solids (35 g/L) was performed in this batch of samples. Again an additional reference sample of 1L of liquid hydrolysate (V47-WSSE-26012023) as used in all the experiments without any solid addition was included in Experiment 2.

Experiment 2 consisted of three individual Samples 4 to 6. As stated above, all samples contained the same concentration of solids (3.4% wt%) suspended in V47-WSSE-26012023 liquid, in particular dried and ground solids from steam exploded wheat straw fibers (WSSE) from batch V40 (Sample 4), non-steam exploded wheat straw fibers from batch Italy (Sample 5) and steam exploded wheat straw fibers (WSSE) from batch Italy (Sample 6). The respective flow diagram of the digestion is depicted in Fig. 3:
On "Day 0", at time 0 h, 50mL of a blend of cellulases (ASA) were added to all samples described above, reaching a final volume of reaction of 1000 ml. At this time point the digestion started at 47°C under agitation in all samples.
On "Days 1 to 3", and particularly at times 24 h, 48 h and 72 h as depicted in Fig. 3, the total volume (1000mL) as well as the digestion parameters (47°C under agitation were not altered. After 72 h of enzymatic digestion, Experiment 3 was terminated.

As detailed in Fig. 3, aliquots were obtained at 0 h, 24 h, 48 h and 72 h. Dry matter contents as well as free mono- and disaccharides concentrations were determined in all aliquots, respectively, as previously detailed.

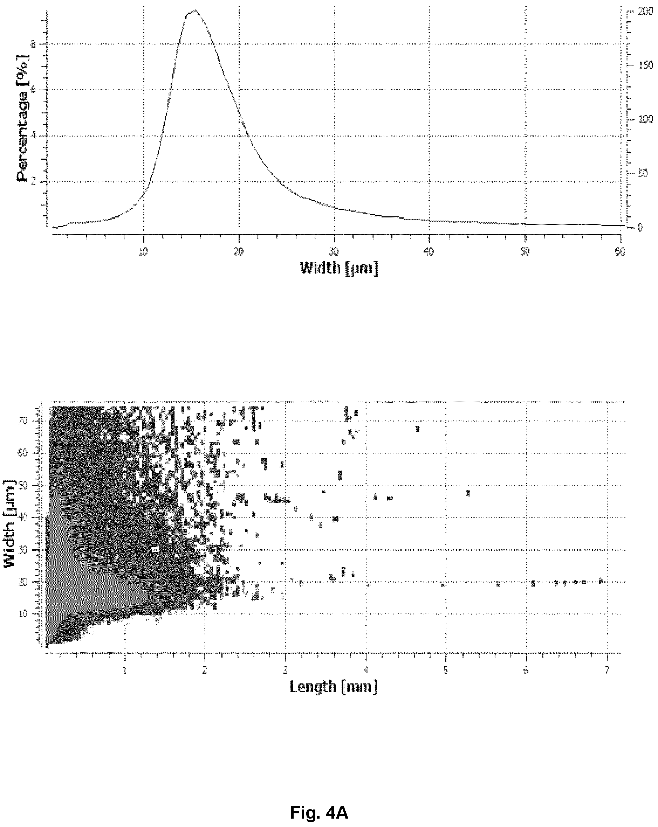

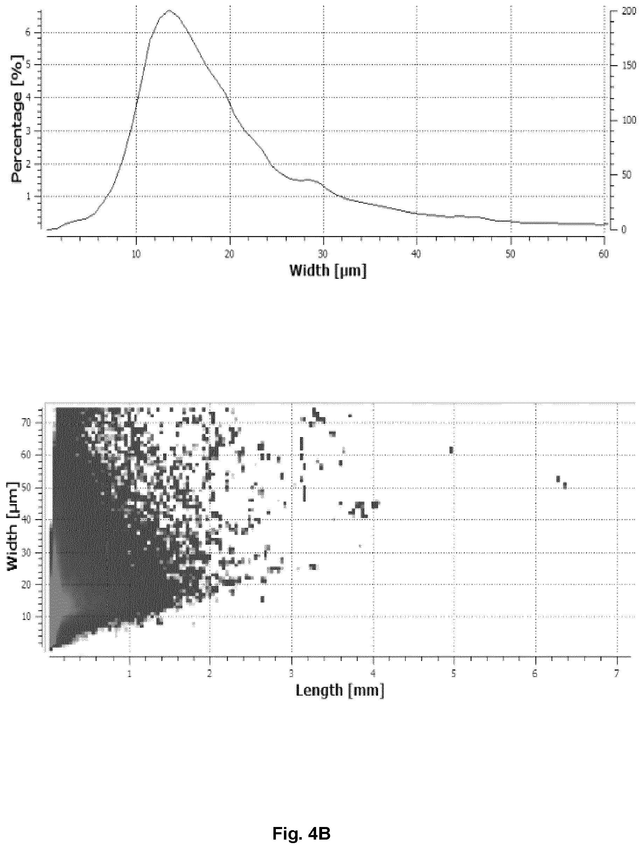

Moreover, a particle size analysis of the substrate used in Experiment 2 was carried out: steam exploded wheat straw WSSE versus non-steam exploded wheat straw WSnonSE). The corresponding results are shown in Figs. 4A and 4B. The specific dimensions (length and width) of the used particles were obtained by an optical analysis using a Fiber Image Analyzer (Valmet FS5, see in this context also US 10 584 298 B2).

Concerning Samples 4 and 6, the following is noted: Solids from wheat straw that were submitted to steam explosion (WSSE) present a content of fines of 85.86 % (particles with length between 0.00 and 0.08 mm). 12.76 % thereof are categorized as "fines A1" having a length up to 0.04 mm. This final product contains 14.14 % of fibers displaying a length-weighted average width of 19.99 µm. The length-weighted average (Lc(I)) and length-weighted average length (ISO. 0.2-7mm) (Lc(I)ISO) of the pulped fibers is 0.555 mm and 0.668 mm, respectively.

Concerning Sample 5, the following is noted: The solids from WSnonSE present a content of fines of 97.76 % (particles with a length between 0.00 and 0.08 mm). 32.37 % thereof are being categorized as "fines A1" having a length up to 0.04 mm. This final product contains 2.24 % of fibers displaying a length-weighted average width of 40.61 µm. The length-weighted average (Lc(I)) and length-weighted average length (ISO. 0.2-7mm) (Lc(I)ISO) of the pulped fibers in the final product is 0.263 mm and 0.432 mm, respectively.

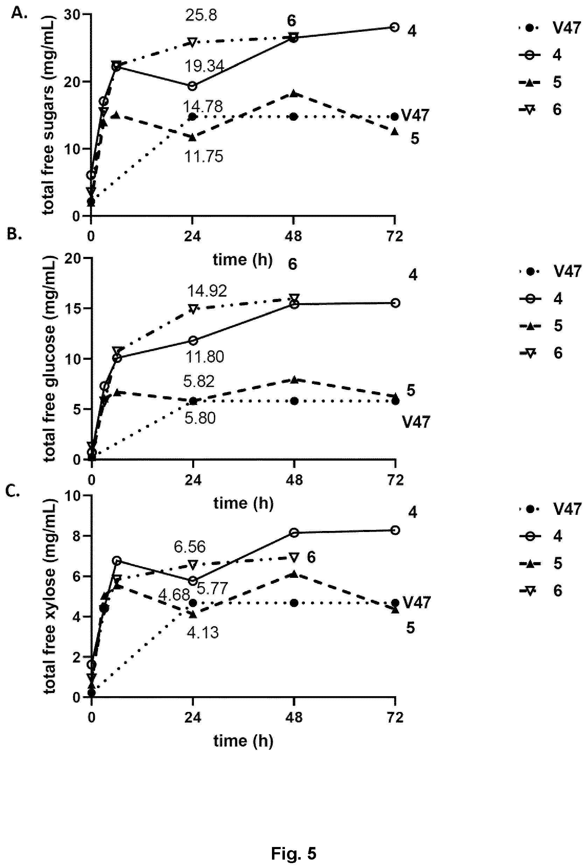

As can be seen from Figs. 4A and 4B, WSSE particles display a two times lower and more homogenous width than nonSE fibers. As such, this parameter "width" appears to have a greater influence on the results of the enzymatic digestion than the length of the respective fibers. The length of the WSSE fibers is about 50 % higher than the length of the WSnonSE fibers, but leads to a two fold increase in free sugars in the liquid fraction after the digestion. In sum, according to the results shown in Fig. 5, it can be concluded that the width of the fiber is the one parameter that correlates better with the yield of the digestion than the length of the solids used as the substrate of the enzymatic reaction.

Fig. 5, and especially charts A to C, show concentration increases of total free sugars (chart A), total free glucose (chart B) and total free xylose (chart C) over a digestion time (h) obtained in an experiment design according to Fig. 3. In Fig. 5, V47 designates the liquid hydrolysate obtained after steam explosion without any fiber addition submitted to the same enzymatic conditions as a negative control. Further, Sample 4 is designated with reference sign 4, Sample 5 is designated with reference sign 5 and Sample 6 is designated with reference sign 6. The following conclusions are drawn:
Fig. 5 shows that after 24 h of enzymatic digestion, there is an increase in the total free sugar concentration of more than twice the original content between WSnonSE fibers (Sample 5) and the WSSE fibers (Samples 4 and 6). Moreover, after 24 h of enzymatic digestion, there is a more than twofold increase of the total free glucose content between the WSnonSE fibers (Sample 5) and the WSSE fibers (Samples 4 and 6).

Furthermore, Fig. 5 shows that, after 24h of enzymatic digestion, a concentration of the total free sugars (chart A) and of the glucose (chart B) in the WSnonSE fibers (Sample 5) is similar to the values obtained in the control sample that was only based on hydrolysate (V47).

Altogether, these results pointed to a need for a steam explosion pre-treatment of the biomass in order to increase the digestibility of the solids and to allow a significant increase of free mono- and disaccharides concentration in the aqueous solution.

Moreover, after 24h of digestion, there is no significant increase in the concentration of total sugars in all samples of Experiment 2. This points to the fact that, after 24h of digestion, all samples seem to have reached a plateau which confirms the results of Experiment 1. Moreover, it indicates that sequential additions of solid substrate at later points in time (such as at 24h and/or at 48h) are needed in order to increase the sugar content in the final product.

Furthermore, Fig. 5 shows that two different batches of wheat straw SE fibers (Samples 4 and 6) present a similar enzymatic digestion profile which renders the methods of the present invention reproducible.

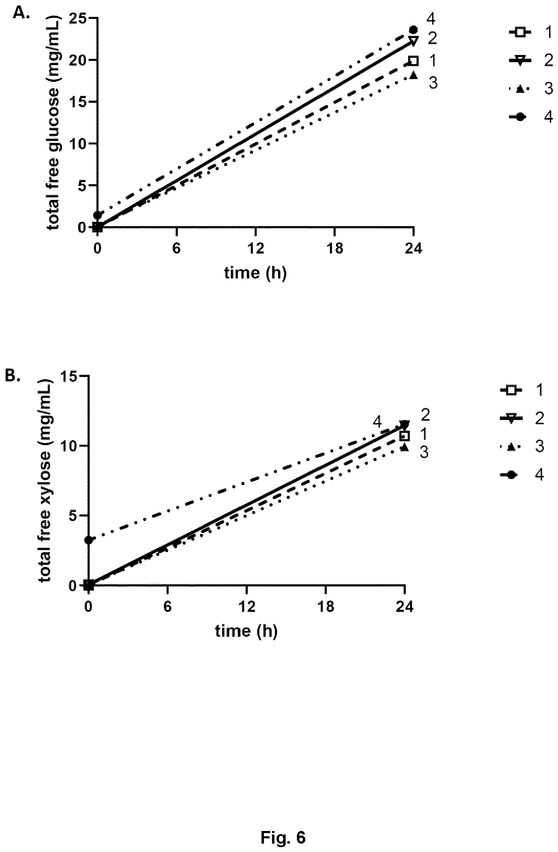

Fig. 6 shows a comparison of specific results from the first and the second experiment with respect to total free sugar, total free glucose and total free xylose concentrations (mg/mL) over time (h), especially comparing Samples 1 to 3 of Experiment 1 with Sample 4 of Experiment 2 over 24 h. Different enzymes were used for Samples 1 to 4: Sample 4: 50 mL or 5 vol% of ASA cellulase; Samples 1 to 3: 10 mL or 1 vol% of Novozymes Cellic Ctec3HS.

Fig. 6 shows that the usage of 5 vol% of ASA cellulase leads to a comparable or even the same final free sugar concentration as the usage of 1 vol% of Novozymes Cellic Ctec3HS. There are no significant differences between Samples 1 to 4, if the value of Sample 4 is corrected by a factor of 2 (Sample 4 starts with half of the fibers content than Samples 1, 2 and 3). This circumstance further reinforces the reproducibility of the method of the present invention. It also shows that once the activity of the enzyme has been titrated, different enzymes can be used without altering significantly the final yield of the reaction.

A pre-hydration of the fibers to be added at 0h does not seem to compromise the digestion process. While it might alleviate the initial high consistency of the suspension when the solids are added in the dried form, at the end it prolongs the process as a whole for at least one more day without any significant gain in sugar concentration.

### Experiment 3: Enzymatic Digestion 3

In the following, a third enzymatic digestion experiment (Experiment 3) is described. Experiment 3 focused on establishing the versatility of an enzymatic digestion process in terms of vegetable biomass origin. For this purpose, it was evaluated whether fibers obtained from canary grass submitted to the same steam explosion process are also able to give high amounts of free carbohydrates during/after enzymatic digestion of the biomass similar to what is observed with wheat straw fibers. In particular, steam exploded, i.e. SE wheat straw fibers or SE cattail grass fibers were added during enzymatic digestion for increasing the sugar content or concentration in the liquid hydrolysates that were obtained after steam explosion.

Dried wheat straw fibers (WSSE fibers) and canary grass fibers (RGSE-12012023, obtained by steam explosion) were used as additional substrate in the following batch of enzymatic digestion experiments falling under Experiment 3. During Experiment 3, three consecutive additions (at 0 h; 24 h and 48 h) of the above defined solids were performed. Moreover, for negative control, a reference sample of 1L of only the liquid hydrolysate V47-WSSE-26012023 (the same used in all the experiments) was prepared.

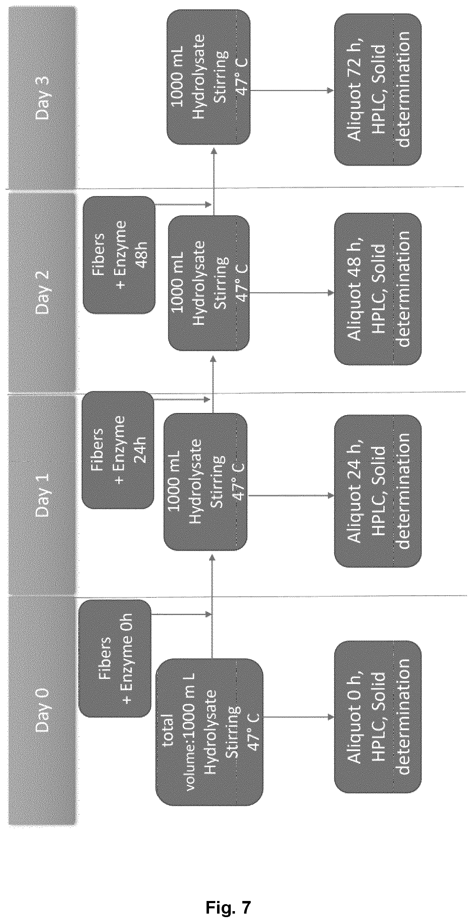

Experiment 3 consisted of three individual samples. Sample 7 used dried wheat straw pulp V40, Samples 8 and 9 used dried and ground reed grass fibers as set out above. Samples 7 to 9 were started with 70g per flask in V47-WSSE-26012023 as hydrolysate. A timeline of Experiment 3 is shown in Fig. 7.

On "Day 0", at time 0 h, the same content of fibers and enzymes (ASA cellulases: 20 mL/L, hydrolysate: V47-WSSE-26012023) were added to all samples. In all cases the total volume remained at 1000 mL of hydrolysate. The digestion was carried out at 47°C under agitation.

After 24h of enzymatic digestion ("Day 1"), an addition of fibers and enzyme (ASA cellulase: 15 mL/L) was performed to each reaction vessel. In particular, the following amounts of dried and ground fibers were added:
Sample 7: 52 g of dried wheat straw pulp V40 (WSSE);
Sample 8: 69.86 g dried and ground canary grass (RGSE) fibers;
Sample 9: 43 g dried and ground canary grass (RGSE) fibers;
As depicted in Fig. 7, the digestion parameters (47°C under agitation) as well as the final reaction volume (1000 mL) were maintained.

After 48 h of enzymatic digestion ("Day 2"), a new amount of fibers and enzyme (ASA cellulase: 15 mL/L) was added to each reaction vessel. In particular, the following amounts of dried and ground fibers were added:
Sample 7: 28.69 g of dried wheat straw pulp V40;
Sample 8: 38.88 g dried and ground canary grass fibers;
Sample 9: 41.09 g dried and ground canary grass fibers.

As depicted in Fig. 7, the digestion parameters (47°C under agitation) as well as the final reaction volume (1000mL) were maintained.

After 72 of enzymatic digestion ("Day 3"), Experiment 3 was terminated. Altogether the total amount of fibers used in each digestion is detailed below:
Sample 7: 150.69 g of dried wheat straw pulp V40 (13 wt%);
Sample 8: 178.74 g dried and ground canary grass (RGSE) fibers (15 wt%);
Sample 9: 154.82 g dried and ground canary grass (RGSE) fibers (13 wt%).

As depicted in Fig. 7, the digestion parameters (47°C under agitation) as well as the final reaction volume (1000mL) were maintained.

As detailed in Fig. 7, aliquots were obtained at 0 h, 24 h, 48 h and 72 h. Dry matter contents as well as free mono- and disaccharides concentrations, respectively, were determined in all the aliquots as previously described.

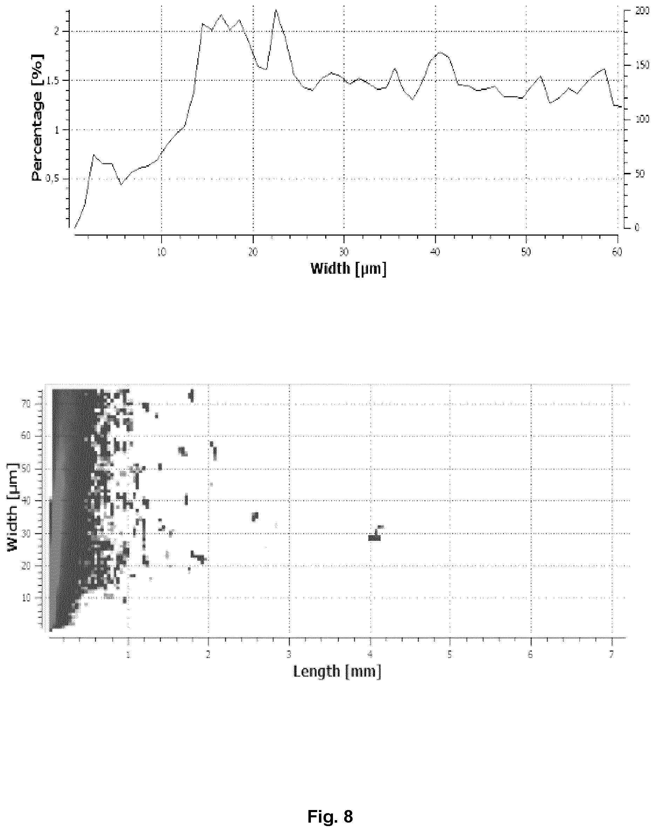

Moreover, before Experiment 3 was started, a particle size analysis was carried out. Figs. 8A and 8B show results of a particle size analysis of both dried and ground substrates, steam exploded wheat straw (WSSE) (Fig. 8A) and steam exploded canary grass (RGSE) (Fig. 8B), used in the Experiment 3. The specific dimensions (length and width) of the used particles were obtained by optical analysis using a Fiber Image Analyzer (Valmet FS5, see in this context also US 10 584 298 B2).

Altogether, the particle size determinations showed the following:
a) Concerning Sample 7: The solids from wheat straw WS that were submitted to steam explosion SE (WSSE) contained an 85.86 % of fines (particles with a length between 0.00 and 0.08 mm). 12.76 % thereof were categorized as "fines A1" having a length up to 0.04 mm. Further, 14.14 % of the dried and ground substrate were identified as fibers displaying a length-weighted average width of 19.99 µm. Their length-weighted average (Lc(I)) and length-weighted average length (ISO. 0.2-7mm) (Lc(I)ISO) were 0.555 mm and 0.668 mm, respectively.
b) Concerning Samples 8 and 9: The solids from RGSE contained 91.72 % of fines (particles with length between 0.00 and 0.08 mm). 58.18 % thereof was categorized as "fines A1" displaying a length lower or equal to 0.04 mm. Further, 8.28 % of the dried and ground substrate were identified as fibers displaying a length-weighted average width of 21.73 µm. Their length-weighted average (Lc(I)) and length-weighted average length (ISO. 0.2-7mm) (Lc(I)ISO) of pulped fibers were 0.533 mm and 0.654 mm, respectively.
c) As can be deduced from Fig. 8, WSSE particles display a narrower width distribution than the one observed for RGSE, but a similar fiber length. As observed by the inventors, this wider width distribution could be related to a less efficient digestion observed in the case of RGSE due to a decreased proportion of substrate being accessible to interact with the enzyme.

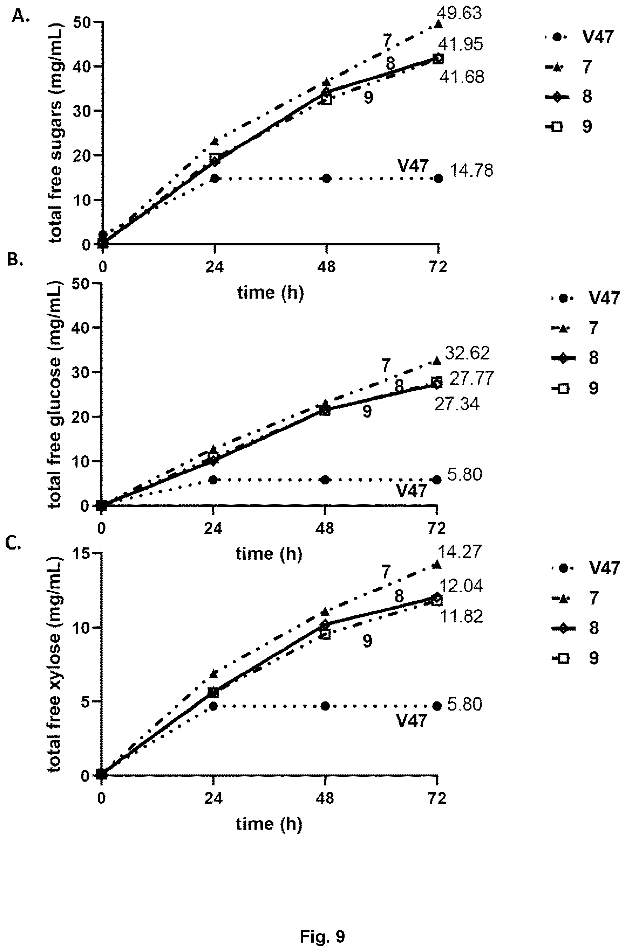

Results shown in Fig. 9, especially charts A to C, describe a concentration increase in total free sugars (chart A), total free glucose (chart B) and total free xylose (chart C) over a digestion time (h) obtained in an experiment design according to a flow chart of Fig. 8.

In Fig. 9, V47 designates the liquid hydrolysate obtained after steam explosion without any fiber addition submitted to the same enzymatic conditions as a negative control. Further, Sample 7 is designated with reference sign 7, Sample 8 is designated with reference sign 8 and Sample 9 is designated with reference sign 9.

Chart A of Fig. 8 shows a more than twofold increase of the concentration of total free sugars when comparing the hydrolysate (V47) values with the respective values obtained when using the above stated two different sources of SE fibers (Samples 7, 8 or 9) after 72h of enzymatic digestion. Furthermore, there is a slight decrease of 16% in total free sugars concentration when comparing the RGSE samples (Samples 8 and 9) with the WSSE sample (Sample 7).

Moreover, the total free glucose concentration in canary grass SE (Samples 8 and 9) digestate is only 16% lower than the respective wheat straw value (Sample 7) (see especially chart B of Fig. 8).

Chart C of Fig. 8 shows, after 72h of digestion, a small decrease of 14% in the total free xylose concentration when using RGSE (Samples 8 and 9) instead of WSSE (Sample 7) as the respective substrate of the digestion. Finally, when calculating the relative mono- and disaccharide composition in each sample at the end of Experiment 3, no significant differences among the samples were observed (Glucose: 65.72 % in Sample 7 vs. 65.90% mean value of Samples 8 and 9; Xylose: 28.74% in Sample 7 vs. 28.53% mean value of Samples 8 and 9).

Experiment 3 indicates that the small reduction in the sugar concentration observed when using dried and ground RGSE fibers as the substrate of the enzymes could be, in part, attributed to the increase in average width of these particles when compared with the values obtained in the case of dried and ground wheat straw steam exploded fibers. These results further confirm the first analysis established after Experiment 1.

In sum, the results presented here evidence a high potential of using RGSE fibers as a substrate for enzymatic activity leading to a controllable increase of total free sugars after only three days, i.e. 72 h of digestion. Moreover, this experiment demonstrates the versatility of the process by proving that it can be applied to different sources of steam exploded fibers.

Based thereon, further Experiments 4 and 5 were performed to confirm the versatility of the method of the present invention regarding the variable origin of the steam exploded fibers used.

### Experiment 4: Enzymatic Digestion 4

In the following experiment (Experiment 4), steam exploded reed grass (Scientific name: *Phragmites*) (RSE) was used as a substrate to be added in the following enzymatic digestion experiments in order to increase the sugar content in the resulting liquid. In this experiment two samples of RSE were used, wherein the substrate was added to the same hydrolysate (V47 -WSSE - 26012023) containing initially 20mL/L of the enzymatic blend (ASA cellulase). All reactions were carried out at 47°C under agitation and final volume of both samples was 1000mL. In order to avoid a great increase in viscosity of the suspension during the digestion, the solid substrate was added in two steps, i.e. 80 g/L at 0h and 70g/L after 24h. The total amount of solids added to each sample was 150g/L (13 % of total mass). After 24h of digestion 15 mL/L ASA cellulase were added to each sample. Experiment 4 was terminated after 48 h.

From this batch of experiments, an increase in the total free sugar concentration of at least 2.4 folds was observed between the samples containing RSE fibers and samples containing the mere hydrolysate (V47) without any substrate upon 48h of enzymatic digestion. No differences were observed between the relative sugar composition (% of total free sugars) of the final RSE samples (Glucose: 50 %. Xylose: 30%). The digestion of RSE fibers led to an average content of 42 g/L of free sugars which is compatible with a good yield of bacteria cell growth.

Furthermore, the total free glucose concentration of Experiment 4 is comparable with the yield obtained when using Wheat straw SE fibers as substrate (see Experiment 2 after 48 h of enzymatic digestion). It is noted that the respective samples obtained in Experiment 4 would need a 6 to 8.5 fold concentration step to reach for instance 300g/L to be compatible with a good yeast growth.

### Experiment 5: Enzymatic Digestion 5

This batch of experiments was specifically aimed at identifying the efficiency of an enzymatic digestion in case the substrate used was pretreated before starting a digestion. For this purpose, three samples were analyzed. In the first sample, dried and ground steam exploded Chinese reed (Scientific name: *Miscanthus*) (MISE) fibers, in the following miscanthus, were employed. In the second sample dried and ground non-steam exploded miscanthus was used as substrate. In the third sample, dried and ground non-steam exploded miscanthus that was submitted to a hot water pre-treatment at 85°C for 60 min was used. In addition, a negative control containing only the hydrolysate and the enzyme was included in Experiment 5. The same hydrolysate (V47) and the same quantity of enzymes were added to each sample. All enzymatic digestion reactions of Experiment 5 were performed in a final volume of 1000mL at 47°C under agitation. To avoid a great increase in the viscosity of the suspension during the digestion, 35 g/L of the solid substrate and 50mL/L of ASA cellulase were added to each sample at time 0h. Aliquots of each reaction were obtained at 0h and at 24h. Experiment 5 was terminated after 24h of enzymatic digestion.

From this batch of experiments the following could be concluded: After 24h of digestion, only the sample that containing steam exploded miscanthus fibers presented a high increase (>30 folds) in total free sugars, glucose or xylose concentration when compared with the negative control, the non-steam exploded or the non-steam exploded and hot water pretreated samples. In addition, similar low total free sugar, glucose and xylose concentrations were observed between the samples containing non-steam exploded miscanthus and the negative control.

The total free sugars concentration detected in the sample containing MISE after 48h of enzymatic digestion was higher than the result observed when steam exploded wheat straw fibers (WSSE, see again Experiment 3) were employed as the enzymatic substrate (WSSE: 20-30 mg/mL vs. MISE: 44mg/mL). Of note is also the relative composition of 57% glucose and 33% xylose in the final product obtained based on steam exploded miscanthus).

In sum, the results obtained in Experiment 5 further highlight the importance of implementing a steam explosion process before starting the enzymatic digestion so as to allow a production of a significantly higher yield of a total free sugar content in comparison to using non-steam exploded fibers.

As being generally applicable to all embodiments of the methods of the invention, the following is noted: While even further steps of filtering may be added, the filtered feedstock derivate(s) may also additionally be centrifuged to properly separate solids that were not digested or filtered from the liquid portion. In addition, it is noted that in the context of the present invention centrifuging is preferred to filtering, but respective combinations may lead to better results than the usage of just one filtering or centrifuging.

In case a concentration step is needed and/or to avoid potential caramelization of free mono- and disaccharides present in the feedstock, a water evaporation should be performed at temperatures lower than 50°C. In addition or alternatively, this process should be performed at reduced pressure.

### Industrial Application

As indicated earlier, examples for biotechnological applications for the present invention may be found in context with bacterial growth and further production of biodegradable bioplastics such as Polylactid Acids (PLAs), Polyhydroxyalkanoates (PHAs), etc. Moreover, such applications may also involve yeast growth and further production of lipids, fatty acids, terpenes and/or products from microorganism metabolism. Other examples may lie in the field of bio combustibles, such as ethanol, biogas etc.

In comparison to known biomass applications, for instance wherein the underlying biomass is not submitted to steam explosion, second-generation feedstock derivatives of the present invention have a significantly increased content of free carbohydrates allowing for a specifically high degree of microorganism proliferation.

Depending on the final application, this increase of free carbohydrates can systematically lead to an increased yield of fermentation products as well as the production of lipids. When using a steam explosion as suggested in the methods of the present invention, the final second-generation feedstock derivatives may comprise an increased concentration of volatile organic acids that can be further used as carbon sources by some microorganisms (i.e. bacteria and/or microalgae) contributing to either an enhanced cell proliferation or a particular enhanced yield of a specifically tailored final product, for instance obtained after cell fermentation. This is particularly interesting for/when increasing propionic acids and/or levulinic acids in the context of specific types of PHAs.

Moreover, the possibility to achieve a specifically low content of solids in the second-generation feedstock derivatives of the present invention (less than 0.1 wt%) facilitates potential upstream processes involved in microorganism culture in fermentation reactors. In this context, it is noted that solids can act as inhibitors of proliferation or fermentation processes or they can interfere with techniques that are used when producing a desired product via microorganisms and/or during cell growth monitoring tests.

Due to the mild conditions used to obtain the second-generation feedstock derivatives of the present invention, none or only small non-inhibitory concentrations of furfural and 5-hydroxyfurfural remain.

The specific methods of the present invention unveil uncounted possibilities of tailoring a feedstock as well as second-generation feedstock derivatives according to uniquely required technical specifications and applications. Parameters in this context are, for instance, depending on specific amounts of total free carbohydrates and/or specific proportions of different mono- and disaccharides in the remaining liquid to be achieved. Key factors may, for instance, lie already in submitting different agricultural by-products to steam explosion as such and/or in (pre-)fabricating material combinations before and/or during enzymatic digestions, and/or the usage of different enzymatic digestion strategies.

The invention has been described with reference to examples and preferred embodiments, wherein the individual features of the described examples and embodiments may be freely combined and/or interchanged, provided that they are compatible. Likewise, individual features of the described embodiments may be omitted, provided they are not absolutely necessary. For the person skilled in the art, numerous variations and embodiments are possible and obvious without thereby abandoning the idea of the invention as stated in the appended claims.

## Claims

**1.** A method of obtaining a solution containing free sugars from a second-generation feedstock, the method comprising the steps of:
providing biomass from a second-generation feedstock;
at least once pre-treating the biomass by steam explosion, the steam exploded biomass comprising a liquid portion and a solid portion;
at least once adding enzymes to the steam exploded biomass to increase an amount of free sugar via enzymatic digestion over a predetermined time, at a predetermined pH value and at a predetermined temperature; and
obtaining the solution by at least partially removing the solids portion from the enzymatically digested biomass.

**2.** The method of Claim 1, wherein the provided biomass from the second-generation feedstock comprises up to 25 wt% of dry matter and up to 75wt% of water before the pre-treatment by steam explosion;
wherein the provided biomass from the second-generation feedstock comprises up to 30 wt% of dry matter and up to 70 wt% of water before the pre-treatment by steam explosion; or
wherein the provided biomass from the second-generation feedstock comprises up to 35 wt% of dry matter and up to 65 wt% of water before the pre-treatment by steam explosion.

**3.** The method of anyone of the preceding claims, further comprising a step of at least once adding further biomass during the enzymatic digestion;
wherein preferably the further biomass is derived from the same and/or from a different biomass than the steam exploded biomass; and/or
wherein preferably the further biomass is pre-treated by steam explosion.

**4.** The method of anyone of the preceding claims, wherein the provided biomass and/or the further biomass is a lignocellulosic biomass and/or a wood-free biomass, respectively;
wherein the provided biomass and/or the further biomass is derived from a renewable biomass, preferably from an annually renewable biomass, respectively;
wherein the provided biomass and/or the further biomass comprises: at least one by-product of a food generation process, agricultural waste and/or at least one agricultural by-product, preferably at least one of wheat straw, tobacco stems, hemp, rice straw, thistle, miscanthus, paludi culture, one or more different types of grasses, cocoa husk, sunflower husk, apple pomace, maize, arundo donax or a combination thereof; and/or
wherein the provided biomass and/or the further biomass is derived from one single source or is a composition of various plants and/or plant parts, respectively.

**5.** The method of anyone of the preceding claims, wherein the step of pre-treating the biomass by steam explosion comprises at least one of the following steps:
steam exploding the biomass under a predetermined amount of pressure, preferably between 7 and 9 bar;
steam exploding the biomass for a predetermined amount of time,
preferably between 10 and 15 min; and
steam exploding the biomass at a predetermined temperature, preferably in the range of 150 and 180°C, even more preferably in a range of 165 to 175 °C.

**6.** The method of anyone of the preceding claims, wherein the predetermined temperature is in a range between 45 to 49 °C, preferably about 47 °C; and/or
wherein the predetermined pH value is in a range between 4.0 and 5.0.

**7.** The method of anyone of the preceding claims, wherein the liquid portion of the steam exploded biomass may be kept or wherein it may be at least partially replaced by any liquid, preferably water, hydrolysate or a mixture thereof before the enzymatic digestion.

**8.** The method of anyone of the preceding claims, further comprising concentrating the obtained solution based on a predefined sugar concentration;
the predefined sugar concentration preferably being a predefined carbohydrate concentration;
the predefined carbohydrate concentration preferably being a monosaccharaide concentration and/or a disaccharide concentration; and/or
the predefined carbohydrate concentration most preferably comprising at least one of the following: glucose, xylose, galactose, mannose, arabinose and cellobiose.

**9.** The method of Claim 8, wherein the predefined glucose concentration is between 50 and 600 g/L.

**10.** The method of anyone of the preceding claims, wherein the steam explosion comprises:
heating the biomass under a predefined pressure to a temperature below 180 °C, preferably to a temperature between 165 to 175 °C, and more preferably for about 10 to 15 min; and
then suddenly releasing pressure.

**11.** The method of anyone of the preceding claims, wherein no chemical products, preferably acids and/or bases, are added during the step of pre-treating.

**12.** The method of anyone of the preceding claims, wherein the step of adding enzymes comprises adding cellulases and/or hemicellulases or a blend thereof.

**14.** The method of anyone of the preceding claims, wherein the step of at least partially removing comprises separating the liquid portion from the non-digested solids portion of the solution by centrifuging and/or filtering; and wherein preferably the step of filtering is a multi-stage filtering.

**15.** The method of Claim 14, wherein the remaining non-digested solids portion is less than 1 wt%; wherein preferably said 1 wt% mainly comprise purified lignin; and/or wherein preferably a total concentration of free carbohydrates lies between 50 to 100 g/L.

**16.** The method of anyone of Claims 14 or 15, further comprising concentrating the centrifuged and/or filtered solution based on a further predefined total content of free carbohydrates, the total concentration of free carbohydrates after the step of concentrating preferably lying between 300 and 600 g/L.

**17.** The method of anyone of the Claims 1 to 15, further comprising a step of concentrating the obtained solution based on a predefined concentration of free carbohydrates, wherein the predefined concentration of free carbohydrates preferably lies between 300 and 600 g/L

**18.** A solution containing at least 5 %or at least 50 to 100 g/L, preferably up to 600 g/L free sugars and obtained by the method of anyone of the preceding claims, the solution comprising carbohydrates, proteins, organic acids, mineral salts and small non-inhibitory concentrations of furfural and 5-hydroxymethylfurfural, preferably below or equal to 0.71 g/L, most preferably in the range of 1.18 and 1.26 g/L for furfural and in the range of 0.3 -0.4 g/L for 5-hydroxymethylfurfural.

**19.** The solution of Claim 18, further comprising lactic acid depending on at least one of the following:
the biomass provided from the second-generation feedstock, the pre-treatment of the provided biomass, the further biomass added during enzymatic digestion and the pre-treatment of the further biomass.

**20.** The solution of Claim 18 or 19, wherein:
the carbohydrates comprise at least one of the following: glucose, xylose, galactose, mannose, arabinose and cellobiose;
the organic acids comprise at least one of the following: acetic, formic, levulinic and propionic; and/or
the mineral salts comprise at least one of the following: sodium, potassium or calcium nitrate, sodium, potassium or calcium phosphates and sodium, potassium or calcium silicates.
